# EUROPEAN PATENT APPLICATION

(11) **EP 1 596 199 A1**
(43) Date of publication of application: **16.11.2005**
(21) Application number: 04011470.4
(22) Date of filing: 14.05.2004
(51) Int. Cl.: G01N 33/68

(54) **Method for the detection of disease-related prion**

(71) Applicant: Prionics AG, CH-8952 Schlieren (CH)
(72) Inventor: Pürro, Mario, 8004 Zürich (CH); Zwald, Daniel, 5430 Wettingen (CH); Schmid, Jaqueline, 8143 Sellenbüren (CH); Biffiger, Karin, 5416 Kirchdorf (CH); Kuhn, Franziska, 8057 Zürich (CH); Oesch, Bruno, 5233 Stilli (CH); Räber, Alex, 8046 Zürich (CH)
(74) Representative: Emmel, Thomas

(57) **Abstract**

Methods for the detection of the disease associated conformation of the prion protein as an indication of transmissible spongiform encephalopathies (TSEs), including preclinical detection of infected live animals and humans, and post-mortem detection methods are disclosed. In one aspect of the invention the tissue or body fluid sample of a test subject is contacted with an antibody that binds only the disease related conformation of the prion protein under non-denaturing conditions.

## Description

### FIELD OF THE INVENTION

The present invention is directed to methods for the detection of prion diseases in animals and humans.

### BACKGROUND OF THE INVENTION

Transmissible spongiform encephalopathies (TSEs) or prion diseases are fatal neurodegenerative diseases in animals and man. The onset of clinical illness is preceded by a long incubation period of months to decades. Clinical symptoms of TSEs include dementia and loss of movement coordination. In the 1980s it was found that a common hallmark of TSEs was the accumulation of an abnormal protease resistant isoform (PrP^{res} or PrP^{Sc}) of the host-encoded prion protein (PrP^{C}) in affected animals and humans. The discovery of PrP^{Sc} provided a molecular marker that was shown to be specific for all prion diseases as well as the major and very likely the only, constituent of the infectious particle, denominated prion.

The protease resistant core of PrP^{Sc}, designated PrP27-30, was discovered by analyzing proteinase K-treated fractions with an apparent molecular mass of 27-30 kDa from Syrian hamster brain for scrapie infectivity. N-terminal sequencing of PrP-27-30 led to the cloning of the host-encoded PrP gene and the identification of the protease-sensitive isoform of the prion protein (PrP^{C}) in uninfected animals. The key pathogenic event in TSEs is the conformational change of the host-encoded prion protein (PrP^{C}) into the pathological isoform named PrP^{Sc} (after its first identification in experimentally scrapie-infected rodents). This conformational change involves refolding of α-helical structures of PrP^{C} into β-sheet of PrP^{Sc}. This refolded prion protein, PrP^{Sc}, differs from PrP^{C} only in its tertiary structure and thus has the same amino acid sequence, as well as the same post-translational modifications such as the N-linked glycosylation and the GPI-anchor. PrP^{Sc} aggregates into amyloid fibrils and accumulates in nervous tissue and to a lesser extent in lymphoreticular tissues.

In an infected host, levels of PrP^{Sc} are directly proportional to prion titers. After experimental inoculation of rodents with TSE agents, PrP^{Sc} is usually detectable in the central nervous system weeks before the appearance of disease, and its level increases to a maximum that is reached up to months before the animal dies.

During the asymptomatic phase, both infectivity and PrP^{Sc} are readily detected in lymphoreticular tissues. Recently, PrP^{Sc} has also been found to accumulate in muscle tissue of hamsters orally infected with scrapie and in experimentally infected transgenic mice.

While the prototype of all prion diseases, scrapie in sheep and goats, has been known for more than two centuries, a new form of animal prion disease designated bovine spongiform encephalopathy (BSE) has since its first recognition in the UK in 1986 developed into a zoonosis. To what extent BSE infected cattle have entered the human food chain is still a matter of debate and has been the subject of a number of studies. A recent study published by the Imperial College in London (Donnelly, C.A., Ferguson, N.M., Ghani, A.C. & Anderson, R.M., *Statistical Methods in Medical Research* **12,** 177-190 (2003)) suggested that based on new epidemiological data up to 1.6 Mio BSE infected cattle could have ended up on the plate of British consumers.

The human prion diseases comprise Creutzfeldt-Jakob disease (CJD), Gerstmann-Straeussler-Scheinker disease (GSS), fatal familial insomnia (FFI) and kuru. These diseases illustrate the three manifestations of prion diseases in general, namely the sporadic forms of the disease (80 - 90% of all CJD cases), the inherited forms linked to mutations in the human PrP gene (familial GSS, familial CJD and FFI) and the infectious forms which are acquired by transplantation, injection or ingestion of prion-contaminated tissue-derived products (iatrogenic CJD, vCJD and kuru). With the emergence of a new form of Creutzfeldt-Jakob disease in the UK in 1996, a new episode in the battle against human diseases caused by food-borne pathogens has been initiated. Up to date, 146 cases of variant CJD (vCJD) have been reported in the UK, and compelling scientific evidence argues for a causal relationship between BSE and vCJD. With no obvious risk factors identified in these patients, a causal link to the exposure of BSE-contaminated food products seems very likely. Detecting vCJD before patients show clinical symptoms is an urgent priority, as it could dramatically reduce the risk of contaminating blood supplies and hospital equipment. Recently, a patient in the UK developed vCJD 6.5 years after receiving a blood transfusion from a donor who developed symptoms ofvCJD 3.5 years after the blood donation (Llewelyn et al., 2004, The Lancet 363, 417-421). This raised the possibility that vCJD might be transmitted by blood transfusion and underscores the need for a diagnostic test to detect prions in blood and fractions thereof. In the present invention a method is disclosed to detect the disease-associated form of PrP in plasma of TSE infected mammals.

Because PrP^{Sc} is the only reliable molecular marker for prion diseases, immunological detection in brain tissue of the protease resistant part of PrP^{Sc} is the basis for rapid diagnostic tests that are currently used for active surveillance of BSE and scrapie. Due to the slow kinetics of accumulation of PrP^{Sc} in the preclinical stage of the disease, the current diagnostic capabilities are strongly limited with respect to the detection of the disease early in the incubation period. The invention disclosed herein provides methods for the detection of prion diseases in the preclinical stage.

While PrP^{Sc} was originally defined as a partially protease resistant and detergent insoluble isoform of PrP, several prion diseases have been identified which are associated with abnormal PrP lacking the classical protease resistance. Forms of PrP that are associated with these prion diseases are termed 'protease sensitive' PrP^{Sc} to distinguish them from the protease resistant forms of PrP^{Sc}.

The detection of the disease related conformation of the prion protein in tissues of an animal or human is thought to be diagnostic of prion disease. To distinguish the disease related PrP from its cellular precursor PrP^{C}, either treatment with proteases to completely destroy PrP^{C}, but only remove a protease-sensitive N-terminal part of PrP^{Sc}, is required, or alternatively an antibody that is able to distinguish between PrP^{C} and PrP^{Sc} can be used. One such antibody that has been shown to react only with native PrP^{Sc} but not with PrP^{C} has been disclosed in our patent application WO 98/37210 and in Korth et al. (1997, Nature 390: 74-77) entitled 'Immunological detection of prions' which is incorporated herein by reference to disclose and describe such antibodies.

Most tests that are currently used for the diagnosis of prion diseases are carried out on tissue of the central nervous system which is obtained *post mortem* from an animal or human either showing clinical disease or suspected of having the disease. The diagnostic tests rely on the use of a pre-treatment to hydrolyse the protease sensitive form of the prion protein followed by the detection of the protease resistant form of the prion protein by an immunological assay. However, such assays will not detect the protease sensitive form of PrP^{Sc}. For example, the patent application WO 00/52197 claims a method to detect PrP^{Sc} in serum of a TSE infected mammal. A major drawback of this method is the use of a pre-treatment with proteases to hydrolyse protease sensitive PrP.

There is an urgent need to provide simple and robust methods for the detection of protease resistant and protease sensitive forms of PrP that are associated with TSEs and diagnostic kits allowing detection of the disease in live mammals.

### OBJECT OF THE INVENTION

It is an object of the present invention to overcome the drawbacks and failures of prior art and to provide methods and diagnostic kits for the detection of prion diseases in live animals and humans.

Such a method would offer the advantage to detect carriers of a transmissible spongiform encephalopathy by screening blood for the presence of disease related PrP and could e.g. prevent vCJD from being accidentally transmitted by blood transfusion.

### DEFINITIONS

The term 'PrP' refers to the common amino acid sequence of the prion protein isoforms rather than to the different conformations.

The term 'PrP^{C}' refers to the prion protein isoform that is abundant in normal mammals and is not related to TSE.

The term 'PrP^{D}' refers to a disease (TSE) related form of PrP which is defined as a conformationally altered isoform of the normal host prion protein. PrP^{D} includes protease resistant and protease sensitive forms of disease associated PrP. The term PrP^{D} is used synonymously: for conformationally altered e.g. ovine, bovine or human PrP, to cite only some examples.

The term 'antibody' refers to any antibody generated by immunization of an animal or by *in vitro* selection/panning procedures. The antibody may be polyclonal or monoclonal, including fragments thereof such as Fab or single chain antibodies. The antibody may be genetically engineered, such as chimeric or humanized.

### SUMMARY OF THE INVENTION

According to the present invention a method is disclosed to detect PrP^{D} in an animal or human comprising taking a sample, preferably blood or other body fluids which are easily obtained from a live mammal, then the sample is brought into contact with an antibody recognizing only PrP^{D}, and finally the amount of antibody/PrP^{D} complex is detected by using an immunological assay.

The method of the invention takes into account that the isoform PrP^{D} related to TSE can be present either as protease sensitive isoform or as protease resistant isoform. In this context it is provided that the sample is treated with a special capture antibody capable of recognizing a conformational epitope on the TSE-related isoform PrP^{D}. The epitope is chosen to be specific for the TSE-related isoform PrP^{D} but not necessarily indicative for the protease resistant isoform of the prion protein. By using a capture antibody recognizing the described conformational epitope one obtains optimal results especially in samples which may contain the TSE-related PrP^{D} alternatively in either the protease sensitive or protease resistant isoform.

In a preferred embodiment the method is used to analyse samples which include PrP^{D} only in the protease sensitive isoform. Such samples can be processed only by using a capture antibody according to the invention.

In a preferred embodiment of the invention the method to detect PrP^{D} is carried out on plasma prepared from a blood sample according to a procedure that is part of this invention.

In another preferred embodiment the plasma sample is treated with chemicals to allow optimal binding conditions for the antibody/PrP^{D} complex.

In another preferred embodiment of the invention the antibody 15B3 is used to detect only PrP^{D} but not the normal form of PrP. The antibody 15B3 is available from Prionics AG, Zurich, Switzerland and methods to generate such antibodies have been disclosed in WO 98/37210. Hybridomacells capable of producing antibody 15B3 have been deposited in connection with WO 98/37210 at DSMZ-Deutsche Sammlung von Mikroorganismen und Zellkulturen GmbH under Accession number: DSM ACC2298.

In yet another preferred embodiment of the invention the antibody/PrP^{D} complex is detected directly or indirectly by an immunological assay.

Another preferred embodiment of the invention provides a method for the detection of the antibody/PrP^{D} complex using a sandwich ELISA with a labelled antibody that recognizes the N-terminus of the prion protein.

A preferred embodiment of the invention is to use the method for the detection of PrP^{D} for testing human blood samples to decide whether the blood is safe for transfusion.

In another preferred embodiment of the invention diagnostic kits are provided for the diagnosis of TSEs in live mammals.

### DETAILED DESCRIPTION OF PREFERRED EMBODIMENTS

According to the invention the method for detection of PrP^{D} in the diagnosis of TSEs comprises
1) collecting a blood sample from an animal or a human
2) preparing plasma from blood sample and treatment of plasma for the binding of the analyte PrP^{D} with the antibody
3) incubating the plasma sample with a PrP^{D} specific antibody as capture antibody
4) washing the antibody/PrP^{D} complex to remove contaminating proteins
5) detecting the antibody/PrP^{D} complex with a sensitive immunological assay

In the following the individual steps will be described in more detail with reference to the specific examples. It is to be understood that the disclosed method and components are not limited to particular samples, chemical agents, antibodies, labels, or assays as such may vary. Unless defined otherwise, all technical and scientific terms used herein have the same meaning as commonly understood by one of ordinary skill in the art to which this invention belongs.

A blood sample is withdrawn from a mammal and placed in a container that contains an anticoagulant such as EDTA, sodium citrate or heparin. A preferred anticoagulant is EDTA but others may be used with similar results. Separation of plasma from the blood cells is performed by centrifugation at 1000xg. Other preferred samples are body fluids such as saliva, urine or cerebrospinal fluid. If serum is used the blood is withdrawn into a container with no anticoagulants and the blood is allowed to clot for at least 30 minutes at room temperature followed by centrifugation at 1000x g to separate the blood clot from serum.

The plasma sample is mixed with an equal volume of buffer containing a detergent to prevent the unspecific binding of PrP^{C} to the antibody. Vast varieties of detergents that are suitable for blocking unspecific binding exist and are known to the person skilled in the art. Preferred detergents are, sodium laurylsarcosine, sodium deoxy cholate, sulfobetaine, Triton X-100, NP-40, ZWITTERGENT, Tween-20, Tween-80. The preferred detergent used in a particular assay format and for a particular species may vary. Preferred concentrations are between 0.05 and 1 %.

The plasma sample is brought into contact with the antibody. In a preferred embodiment of the invention an antibody that is specific for the disease associated form PrP^{D} is used. A preferred antibody is the antibody 15B3 which has been shown to be specific for PrP^{D} but other antibodies may be used in a similar fashion. In a particularly preferred embodiment the antibody is coated on the wells of a microtitre plate or to beads to capture PrP^{D} in the sample. The complex of the antibody with PrP^{D} is then detected in an immunological assay using a detection antibody that recognizes PrP. Detection can be achieved by any immunological method. Such assays are well known to those skilled in the art and may include Western blotting (immunoblotting), enzyme linked immunosorbent assays (ELISA), immuno-PCR or fluorescence activated cell sorting (FACS). Direct detection may be carried out by using an antibody to the prion protein that is conjugated with an enzyme such as peroxidase, alkaline phosphatase or fluorescent molecules or nucleic acids. Alternatively the detection can be indirectly using an unlabelled primary (anti prion protein antibody) and a labelled secondary antibody.

The invention is illustrated in the following non-exclusive examples and accompanying figures:

### EXAMPLE 1: Determination of PrP^{D} in plasma from BSE negative and positive cattle

### EXAMPLE 2: Determination of PrP^{D} in plasma from scrapie negative and positive sheep

### BRIEF DESCRIPTION OF THE DRAWINGS

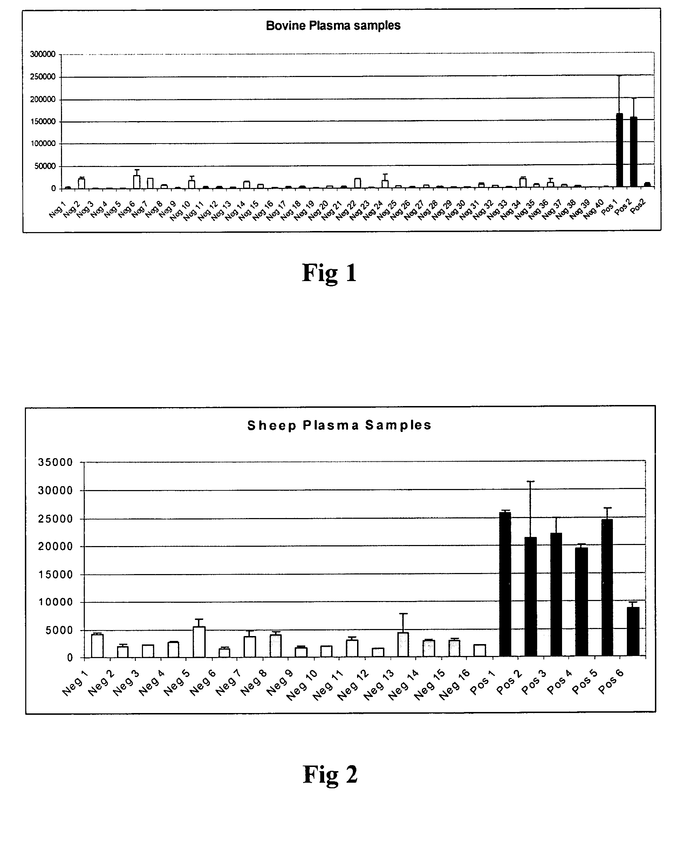

Figure 1 is a graph showing the results of Example 1. In this example a sandwich immunoassay was performed using 15B3 as a capture antibody and an antibody recognizing the N-terminus of PrP as a detection antibody on plasma samples from BSE infected (Pos1-Pos2) versus normal cows (Neg1-Neg40).
Figure 2 is a graph showing the results the results of Example 2. In this example a sandwich immunoassay was performed using 15B3 as a capture antibody and an antibody recognizing the N-terminus of PrP as a detection antibody on plasma samples from scrapie infected (Pos1-Pos6) versus normal sheep (Neg1-Neg16).

### EXAMPLE 1

The graph in Figure 1 shows the results of a first experiment. The data shown illustrate that BSE positive plasma samples can be distinguished from negative samples using the sandwich immunoassay. The mean of double measurements are shown whereas the error bars indicate the difference to the higher value.

96-well plates were coated with 2µg/ml anti-IgM antibody diluted in phosphate buffered saline (PBS) pH 7.4, 0.1% bovine serum albumin (BSA) for 1 hour at room temperature. Unbound antibody was removed by washing the plates three times with PBS pH 7.4 containing 0.05% Tween-20. Thereafter the plates were blocked for 2 hours and washed three times with PBS pH 7.4 containing 0.05% Tween-20. Conformation specific antibody 15B3 in PBS pH 7.4 at a concentration of 2µg/ml was bound to anti-IgM for 1hour at room temperature. After washing the plate three times with PBS pH 7.4 containing 0.05% Tween 20, 110 µl Plasma from BSE negative and positive cattle diluted 1:1 with Tris buffered saline (TBS) containing 0.4% deoxycholate (DOC) was added to the plate and incubated for 1.5 hours at room temperature. Unbound proteins were washed away with TBS containing 0.2% DOC. Peroxidase labelled monoclonal antibody 805 recognizing amino acids 25-40 of PrP was diluted to 10ng/ml in a buffer containing PBS pH 7.4, 0.02% Casein, 0.1% Tween-20 and added for 1 hour to wells. Unbound antibody was removed by washing for three times with PBS pH 7.4 containing 0.05% Tween-20. 100 ml of chemiluminescent substrate solution was added to the wells and read in a plate luminometer. The values are given as relative light units.

### EXAMPLE 2

The graph in Figure 2 shows the results of a second experiment. The data shown illustrate that Sheep scrapie positive plasma samples can be distinguished from negative samples using the sandwich immunoassay. The mean of double measurements are shown whereas the error bars indicate the difference to the higher value.

Coating of plate with 15B3 antibody was performed as described in example 1. 110 µl of Plasma from scrapie negative and positive sheep diluted 1:1 with TBS containing 0.2% Sarcosyl was added to the plate and incubated for 1.5 hours at room temperature. Unbound proteins were washed away with TBS containing 0.1 % Sarcosyl. POD labelled monoclonal antibody 805 recognizing amino acids 25-40 of PrP was diluted to 10ng/ml in a buffer containing PBS pH 7.4, 0.02% Casein, 0.1% Tween-20 and added for 1hour to wells. Unbound antibody was removed by washing for three times with PBS pH 7.4 containing 0.05% Tween-20. 100 µl of chemiluminescent substrate solution was added to the wells and read in a plate luminometer. The values are given as relative light units.

## Claims

1. Method for the detection of an isoform (PrP^{D}) of the prion protein in body fluids and fractions of body fluids, especially blood or urine, wherein the isoform PrP^{D} is related to TSE and is present as either protease sensitive isoform or as protease resistant isoform, the method comprising:
a. treatment of the body fluid or its fraction with a capture antibody capable of recognizing a conformational epitope on the TSE-related isoform PrP^{D}, said epitope being specific for the TSE-related isoform PrP^{D} but not necessarily indicative for the protease resistant isoform of the prion protein and
b. detecting possible PrP^{D}-capture antibody complexes.

2. Method according to claim 1, wherein the body fluid includes PrP^{D} only as protease sensitive isoform.

3. Method according to claim 1 or 2, wherein the body fluid or its fraction is treated with an additional detection antibody capable of recognizing the N-terminus of prion-protein (PrP).

4. Method according to one of the preceding claims, wherein the method to detect PrP^{D} is carried out on plasma prepared from a blood sample.

5. Method according claim, wherein the plasma sample is treated with chemicals to allow optimal binding conditions for the antibody/PrP^{D} complex.

6. Method according to one of the preceding claims, wherein the antibody 15B3 is used as capture antibody.

7. Method according to one of the preceding claims, wherein the anti-body/PrP^{D} complex is detected directly or indirectly by an immunological assay.

8. Use of a method according to the preceding claims for testing blood or blood fractions, especially in connection with transfusion of human blood.
